# EUROPEAN PATENT APPLICATION

(11) **EP 3 639 730 A1**
(43) Date of publication of application: **22.04.2020**
(21) Application number: 18200603.1
(22) Date of filing: 16.10.2018
(51) Int. Cl.: A61B 5/00

(54) **SUPPLY OF A SENSOR OF AN INTERVENTIONAL DEVICE**

(71) Applicant: Koninklijke Philips N.V., 5656 AG Eindhoven (NL)
(72) Inventor: MUELLER, Manfred, 5656 AE Eindhoven (NL); VAN DER HORST, Arjen, 5656 AE Eindhoven (NL)
(74) Representative: de Haan, Poul Erik

(57) **Abstract**

The present invention relates to the supply of a sensor of an interventional device. In order to provide an interventional device with improved handling, an interventional device is provided, the device comprising a longitudinal elongated main body with a distal portion and a proximal portion; and a sensor provided on the distal portion. The elongated main body comprises a hollow shaft. The proximal portion of the main body comprises an optical energy generation section, in which the hollow shaft is at least partially provided as a transparent hypotube, and in which a doped material is provided inside the hollow shaft. Further, the doped material is configured to generate light as stimulated emission with a predetermined wavelength upon the doped material being radiated with a pumping wavelength. Still further, the transparent hypotube is configured to receive light from an external light source as a substantially transversal light input providing the pumping wavelength to the doped material. The main body further comprises a light guiding section that comprises an optical fiber arrangement inside the hollow shaft extending from the optical energy generation section toward the sensor for transmitting energy to the sensor.

## Description

### FIELD OF THE INVENTION

The present invention relates to the energy supply of and communication with a sensor of an interventional device, and relates in particular to an interventional device for intravascular application, to an interventional system and to a method for supplying optical energy to a sensor of an interventional device.

### BACKGROUND OF THE INVENTION

In the medical field, guidewires are used for different types of interventions and examination procedures. A guidewire is inserted into a body of a subject, for example into the vascular structure. This may be done under observation by imaging methods like X-ray fluoroscopy. Once the guidewire is placed and the tip has reached the target location, the guidewire can also act as guide for further steps, like inserting devices like stents or other prosthesis along the guidewire. During placement of the guidewire, sensors on the guidewire can be used to provide data, for example data useful for navigation within a vascular structure. For example, US 2014187982 A1 describes an intravascular device with sensing components in which wireless communication is provided. However, it has been shown that wire connections for energy supply of the sensors or the like may be cumbersome in handling of the guidewire.

### SUMMARY OF THE INVENTION

There may thus be a need to provide an interventional device with improved handling.

The object of the present invention is solved by the subject-matter of the independent claims; further embodiments are incorporated in the dependent claims. It should be noted that the following described aspects of the invention apply also for the interventional device for intravascular application, for the interventional system and for the method for supplying optical energy to a sensor of an interventional device.

According to the present invention, an interventional device for intravascular application is provided. The device comprises a longitudinal elongated main body with a distal end and a proximal portion and a sensor provided near the distal end, on a distal portion. The elongated main body comprises a hollow shaft. Further, the proximal portion of the main body comprises an optical energy generation section, in which the hollow shaft is at least partially provided as a transparent hypotube, and in which a doped material is provided inside the hollow shaft. Still further, the doped material is configured to generate light as stimulated emission with a predetermined wavelength upon the doped material being radiated with a pumping wavelength. The transparent hypotube is configured to receive light from an external light source as a transversal light input providing the pumping wavelength to the doped material. Furthermore, the elongated main body further comprises a light guiding section that comprises an optical fiber arrangement inside the hollow shaft extending from the optical energy generation section to the sensor for guiding light to the sensor.

This provides a facilitated handling, since device itself does not need cables or the like.

According to an example, the light generated by the optical energy generation section is provided i) for communicating with the sensor via light modulation and/or ii) for supplying the sensor with energy provided by the guided light.

The light source can be supplied via a cable or via an integrated battery.

According to an example, it is provided a first mirror element at a distal transition portion of the optical energy generation section with the doped material to the optical fiber arrangement in the hollow shaft of the light guiding section. It is further provided a second mirror element at a proximal end portion of the optical energy generation section with the doped material. The first mirror element is partly reflective for the generated light with a first reflective grade, and the second mirror element is reflective for the generated light with a second reflective grade being higher than the first reflective grade.

According to an example, the sensor provides measurement results as optical signals that are guided by the optical fiber arrangement. A light scattering section is provided at the proximal portion that comprises a light scattering material to provide the optical signals as scattered light for detection by an external signal detector.

According to an example, the interventional device is at least one of the group of a guidewire, a catheter and a needle.

According to an example, provided in addition or alternatively, the sensor is at least one of the group of a pressure sensor, an imaging sensor (ultrasound, OCT), a temperature sensor, a pH-sensor, a biomarker sensor, a chemical sensor, a flow volume sensor, a light absorption sensor, a light scattering sensor and a flow velocity sensor.

According to an example, the tip of the device contains a photodiode and an electrical sensor, wherein the photodiode transfers optical energy into electrical energy to power the sensor.

According to the present invention, also an interventional system is provided. The system comprises an interventional device for intravascular application according to one of the preceding examples and an external light source. The external light source is provided to supply a transversal light input to the transparent hypotube providing the pumping wavelength to the doped material.

According to an example, the light source is provided as a detachable light source.

According to an example, the external light source is provided integrated with a vascular access port of the interventional device.
to an example, an external signal detector is provided that detects light emitted from the light scattering section.

According to the present invention, also a method for supplying energy to a sensor of an interventional device is provided. The method comprises the following steps:
a) providing an interventional device for intravascular application, the device comprising: a longitudinal elongated main body with a distal end and a proximal portion; and a sensor provided near the distal end;
b) generating a pumping light with a pumping wavelength and coupling the pumping light as transversal light input into a hollow shaft provided as a transparent hypotube at the proximal portion, inside which hollow shaft a doped material is provided;
c) stimulating the doped material with the light with the pumping wavelength and thereby generating light as stimulated emission with a predetermined wavelength; and
d) transmitting the light by an optical fiber arrangement inside a hollow shaft of the elongated main body to supply energy to the sensor.

According to an aspect, a system comprises an elongated intravascular device, such as a guidewire or micro-catheter, with at least one integrated optical fiber connected to a sensor. Further, an external connector device, e.g. an access port, or a catheter, or a sheath, is provided that partly surrounds a certain length of the intravascular device. The intravascular device can slide (mostly) freely along or through the access device. The external connector device contains a light source, e.g. light diodes, which emit light with a wavelength around *λP*. The access port is optically shielded. The pumping light may be incoherent. The optical fiber inside the intravascular device is doped with absorbers that absorb the light with the wavelength *λP* and re-emit light at a different wavelength *λ1*. The external connector device provides data transfer either via cable or wirelessly and power (via batteries or power cable).

Optionally, the system also incorporates at least one reflector inside the optical fiber forming a laser cavity inside the optical fiber so that the optical fiber and the light source in the external connector device form an optically pumped laser. In a further option, the sensor is an optical sensor or an electrical sensor. In the latter part, a photodiode is provided at the end of the optical fiber to absorb the light and to power the electrical sensor. In another option, the sensor emits coded or modulated light at a wavelength *λ2* back into the optical fiber to communicate the measurement data back to the external connector device. The light with the wavelength *λ2* coming from the sensor can be intentionally scattered out of the fiber at the proximal end of the intravascular device to be detected by e.g. a camera or a photodiode. The pump diodes can be modulated to communicate with (an ASIC at) the sensor. That way one can enable high bandwidth communication between the sensor and a console.

For example, the console is part of a system for controlling the operation of the intravascular device. Hence, the console may be provided as a device or system with which the sensor ultimately communicates with, e.g. directly or indirectly.

In an example, the elongated intravascular device is assumed to be a guidewire with a shaft comprising a hypotube surrounding at least one optical fiber. There might be other elements present, like a core, wiring etc. which are not further shown. The optical fiber is connected to the sensor. The sensor can be an optical sensor, or it can be an electric sensor that this powered via a photo-electric element. In the latter case the fiber forms part of an "optical link".

At least a part of the hypotube is transparent, allowing light from outside to reach part of the optical fiber. At least a part of this optical fiber is doped, e.g. with rare-earth elements like erbium, ytterbium, neodymium, dysprosium, praseodymium, thulium or holmium, to form an active laser medium that can absorb light of a pumping wavelength around *λP* and emit light of a longer wavelength around *λ1* via stimulated emission. The doped region of the fiber is bound by two mirrors forming a laser cavity. The proximal mirror can be just the surface of the cleaved fiber or it can be Fiber Bragg Grating (FBG) reflector. The distal mirror can be a FBG reflector. The reflectivity of the distal mirror should be less than the reflectivity of the proximal mirror.

In an example, not all of the fibers surrounded by the transparent hypotube are doped. In a further example, most of the fibers surrounded by the transparent hypotube are doped. In a still further example, all fibers surrounded by the transparent hypotube are doped.

In an example, the external connector device is assumed to be (part of) a vascular access port. A vascular access port could be a guiding catheter, hemostatic valve, introducer sheath etc. The guidewire is inserted through an opening of the vascular access port into the blood vessel. Since the inner diameter of the port's opening is (slightly) larger than the external diameter of the guidewire, the guidewire can be moved freely through the port's opening. Instead of forming part of a vascular access port, the external connector device can be integrated into other devices that (partly) surround the guidewire, like a guide catheter or a separate device. The external connector device contains light sources proving light of the pumping wavelength *λP*. The light source is preferably an array of pumping diodes. The light source can be continuous wave, flashed, or modulated. Preferably, the pumping light is incoherent, i.e. no laser light. The external connector device is designed in such a way that when the guidewire is inserted into the external connector device, the pumping light will be directed onto the doped fiber inside the guidewire. This can be done by e.g. using mirrors surrounding the light sources. The external connector device will also shield the light source so that little or no light escapes to the outside. To provide communication to the sensor, the external connector can change the light modulation. Communication between the external connector and the console can be wired or preferably wireless. The external connector can comprise an internal battery or can be wired to an external power source. Having wires to/from the external connectors is acceptable, because unlike the guidewire, the connector (e.g. in the form of a vascular access port) is not moved or handled during a procedure.

Together, the doped fiber and the external connector form an optically pumped fiber laser. The pumping light from the diodes is absorbed by the dopants which will then re-emit at *λ1* due to stimulated emission. Because the dopants are inside a laser cavity, nearly all of the light at *λ1* will be emitted into the optical fiber. This makes the arrangement efficient.

In an example, the fiber may be coiled to increase interaction length with the pumping light and therefore absorption efficiency. The pumping light may be modulated to communicate with the sensor.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 schematically shows an example of an interventional device for intravascular application.
Fig. 2 shows another example of an interventional device for intravascular application.
Fig. 3 shows a further example of an interventional device for intravascular application.
Fig. 4 shows an example of an interventional system.
Fig. 5 shows basic steps of an example of a method for supplying optical energy to a sensor of an interventional device.

### DETAILED DESCRIPTION OF EMBODIMENTS

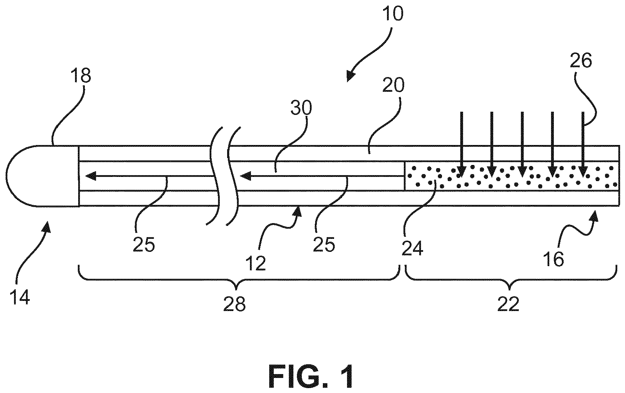

Fig. 1 shows an example of an interventional device 10 for intravascular application. The device 10 comprises a longitudinal elongated main body 12 with a distal end 14 and a proximal portion 16. The device 10 further comprises a sensor 18 provided near the distal end 14, in a distal portion of the elongated body 12. The main body 12 comprises a hollow shaft 20. The proximal portion 16 of the main body 12 comprises an optical energy generation section 22, in which the hollow shaft 20 is at least partially provided as a transparent hypotube, and in which a doped material 24 is provided inside the hollow shaft 20. The doped material 24 is configured to generate light 25 as stimulated emission with a predetermined wavelength upon the doped material 24 being radiated with a pumping wavelength. The transparent hypotube is configured to receive light from an external light source as a transversal light input 26 providing the pumping wavelength to the doped material 24. The light input 26 is indicated with a plurality of arrows. The main body 12 further comprises a light guiding section 28 that comprises an optical fiber arrangement 30 inside the hollow shaft 20 extending from the optical energy generation section 22 to the sensor 18 for guiding light to the sensor 18.

It is noted that in Fig. 1, the distal end 14 is shown in a schematic manner. It is indicated that a catheter tip is provided with an atraumatic (e.g. round) shape suitable for insertion and navigation inside a vessel structure.

As an example, the light generated by the optical energy generation section 22 is provided for communicating with the sensor 18 via light modulation. In addition, or alternatively, the light generated by the optical energy generation section 22 is provided for supplying the sensor 18 with energy provided by the guided light.

The hollow shaft is provided as a hypotube, which may be opaque along the main portion, and which is transparent in the energy generation section.

The term "near" the distal end relates to a location directly in the distal end section, e.g. within the last 15 cm, e.g. within the last 10 cm or last 5 cm.

The hollow shaft part that is provided as the transparent hypotube with the doped material for generating light, is rigidly and permanently connected to the part of the hollow shaft with the optical fiber arrangement inside the shaft for guiding light.

Since the light to be supplied to the sensor is generated inside the hollow shaft and then guided along the shaft, a precise coupling of light into the hollow shaft is thus not necessary.

The doped material 24 is irradiated by the pumping light from the external light source. The light of e.g. pumping diodes excites the doped material. The doped material is activated into a higher state and by falling back to a lower state, light is generated.

For example, the doped material 24 is of rare earth elements comprising erbium and ytterbium ions.

The doped material inside the hollow shaft provides an integrated in-coupling of light, i.e. a coupling of light into the optical fibers.

As a result, a freedom-wire-like experience for guidewires with an optical sensor is provided.

The external light source creates a secondary light beam, and the doped material creates a primary light beam.

The internal coupling of the light works for any position of the guidewire.

For receiving the light, in an example, the transparent hypotube is configured to be in close contact with the external light source as the transversal light input providing the pumping wavelength to the doped material.

For receiving the light, in another example, the transparent hypotube is configured to be functionally connected with the external light source as the transversal light input providing the pumping wavelength to the doped material 24. As an example, the connection is provided as a temporarily attachment, like a detachable mounting of the external light source.

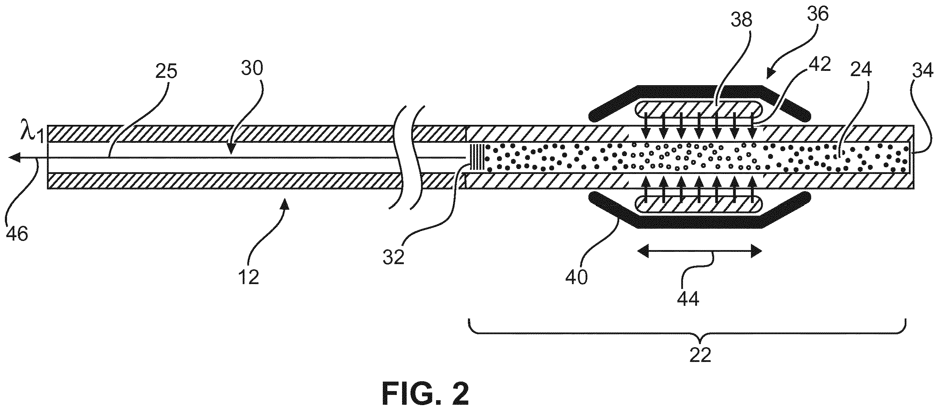

Fig. 2 shows another example of the interventional device 10 for intravascular application. It is further provided a first mirror element 32 at a distal transition portion, where the optical energy generation section 22 with the doped material 24 is ending and the optical fiber arrangement 30 in the part of the hollow shaft of the light guiding section 28 begins. It is still further provided a second mirror element 34 at a proximal end portion of the optical energy generation section 22 with the doped material 24. The first mirror 32 element is partly reflective for the generated light with a first reflective grade. The second mirror element 34 is reflective for the generated light with a second reflective grade being higher than the first reflective grade.

The first and second mirror 32, 34 enhance the pumping effect of the light generated by the doped material 24.

For example, at least the first mirror element 32 is provided as a Fiber Bragg Grating (FBG) reflector. In an example, the second mirror 34 is a cleaved mirror.

It is noted that depending on the degree of the optical dopant, i.e. the doped material 24, and the power of the pumping radiation, the supply of the sensor 18 is also possible without the use of such mirrors and/or filters.

The first and second mirror elements 32, 34 define a cavity that provides a resonator for the light generation. This results in an increase of effectiveness.

Fig. 2 shows an external transversal light source 36 as an option. The external transversal light source 36 may be provided as part of the interventional device 10, but the external transversal light source 36 may also be provided separate, i.e. in addition to the interventional device 10. In other words, the interventional device 10 is also provided without the shown external transversal light source 36. The transversal light input 26 is then provided from another light source.

As an example, the external transversal light source 36 comprises a plurality of pumping light emitting diodes 38 that are arranged at least along a part of circumferential section of the transparent hypotube. Further, as an option, a shielding 40 is provided that optically shields the outside from the light of the pumping light emitting diodes 38. The light emitting diodes 38 supply pumping light 42, indicated with a plurality of arrows, to the doped material 24 inside the hollow shaft 20.

In an example, the external transversal light source 36 is having a ring-shaped cross-section surrounding the elongated intravascular device. In another example, the external transversal light source 36 is having an open C- or U-shaped cross-section such that the elongated intravascular device can be inserted. A releasable closing section may be provided to ensure a proper attachment to the elongated intravascular device.

The external transversal light source 36 is provided to be slidable along the elongated main body 12 as indicated with a double arrow 44. In an alternative embodiment the external light source is attachable to the outer surface of the elongated main body.

In an example, the external transversal light source 36 is provided with integrated batteries for electric power supply. The external light source 36 can therefore be an autonomous energy source, in form of a sleeve that can be slid on the optical energy generation section 22 of the interventional device. In another example, the external transversal light source 36 is provided with cable wire connection for the electric power supply. In an option, batteries and a wire connection are provided in combination.

An arrow 46 indicates the light generated by the doped material 24 passing the first mirror element 32, the light guided towards the sensor 18 (not shown in Fig. 2) having a wavelength around *λ1*.

In a further example, also not shown in detail, multiple fibers are going to multiple sensors. The light going to the sensors is the same for all fibers because of the pumping process, but the light going back (if provided as an option) will be different modulated with the individual signals from each sensor.

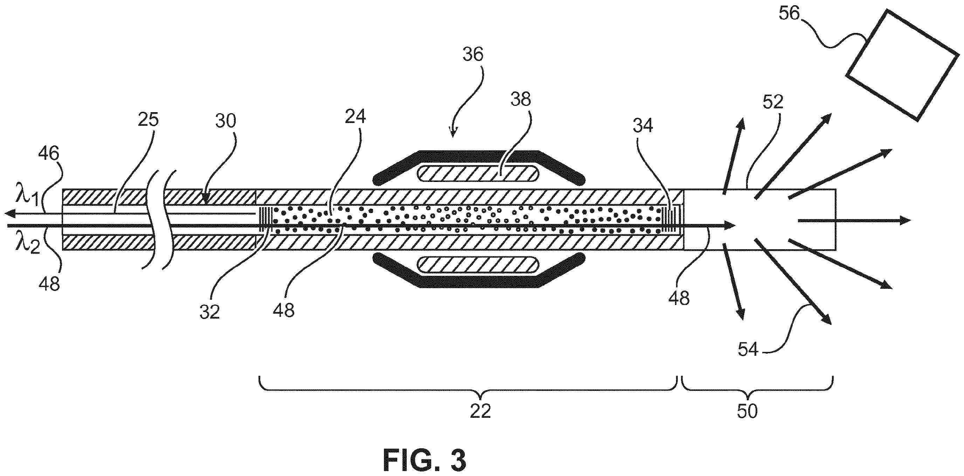

Fig. 3 shows a further example of the interventional device 10 for intravascular application. As an option, the sensor 18 (not shown, provided to the left of the figure) at the distal portion of the interventional device provides measurement results as optical signals 48 (illustrated with a simplified single arrow) that are guided by the optical fiber arrangement 30. A light scattering section 50 is provided at the proximal portion of the interventional device that comprises a light scattering material 52 to provide the optical signals as scattered light 54 for detection by an external signal detector 56.

In such example, a bi-directional communication with the sensor 18 is enabled. The sensor 18 sends light back at the wavelength *λ2*, indicated with ref. numeral 48. This light is modulated with the measured data. In an example, *λ1* ≠ *λ2.* The proximal mirror, i.e. the second mirror element 34 is also a FBG reflector. For example, it is not reflective for *λ2.* In an example, the distal mirror, i.e. the first mirror element 32 is not reflective for *λ2.*

The fiber ends proximally in a scattering element provided by the scattering material 52 that scatters *λ2.* As an example for the external signal detector 56, a photodetector can pick up the scattered light from which the measurement data is extracted. In an example, the external signal detector 56 is not in contact with the wire. The external signal detector 56 may also be integrated into the connector device, from which it can be forwarded wirelessly or by wire to the console, or directly in the console, or it can be a separate (wired or wireless) device. In an example, the distal mirror is not reflective for *λ2.*

The light scattering material provides an integrated out-coupling of light to external use.

In an example, the light scattering section is provided as detachable section at a proximal end of the main body.

In an example, the light generated by the stimulated emission by the doped material 24 is provided with a first frequency range, and the optical signals 48 from the sensor 18 are provided with a second frequency range. The first frequency range is different from the second frequency range.

In an example, the first frequency range is adjacent to the second frequency range without overlapping. In an example, the first frequency range is separated from the second frequency range by a gap range.

In another example, the first and the second mirror elements 32, 34 are each reflective for the optical signals with a third reflective grade being smaller than the first and the second reflective grades. As a result, the optical signals can pass the first and the second mirror elements 32, 34 and can reach e.g. the light scattering section 50.

In an example, the external signal detector 56 may take a similar sleeve-like form as the external light source 36. In a further option the external signal detector may be an autonomous battery powered sleeve, configured to communicate wirelessly with a console that further processes the measurement data collected by the one or multiple sensors located on the interventional device. In a further optional embodiment, the external light source 36 and the external signal detector 56 are integrated in a single battery powered autonomous sleeve, detachable and/or slidable over the optical energy generation section 22 and the light scattering section 50, respectively. The measurement data read out by the external signal detector portion of the sleeve is communicated wirelessly to the console for further processing of the measurement data.

In an example, the interventional device 10 is at least one of the group of a guidewire, a catheter and a needle.

As an option, provided in addition or alternatively to the different device types mentioned above, the sensor 18 is at least one of the group of a pressure sensor, an imaging sensor (ultrasound, OCT), a temperature sensor, a pH-sensor, a biomarker sensor, a chemical sensor, a flow volume sensor, a light absorption sensor, a light scattering sensor and a flow velocity sensor. It is noted that the different types of the interventional device are provided as an additional or as an alternative feature to the different types of the sensor. Hence, in an example, the interventional device is at least one of the group of a guidewire, a catheter and a needle. Further, in addition or alternatively, the sensor is at least one of the group of a pressure sensor, an imaging sensor (ultrasound, OCT), a temperature sensor, a pH-sensor, a biomarker sensor, a chemical sensor, a flow volume sensor, a light absorption sensor, a light scattering sensor and a flow velocity sensor.

The interventional device may be provided as micro-catheter.

In an example, not shown in detail, the tip of the device contains a photodiode and an electrical sensor. The photodiode transfers optical energy into electrical energy to power the sensor 18.

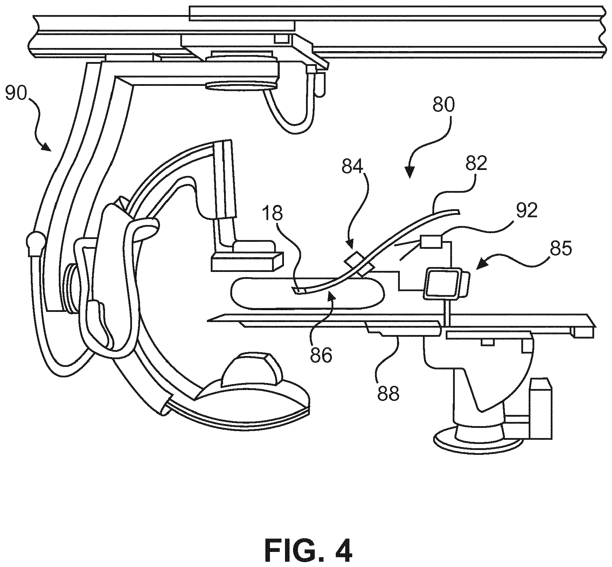

Fig. 4 shows an example of an interventional system 80 that comprises an interventional device 82 for intravascular application according to one of the preceding examples. The system 80 further comprises an external light source 84. The external light source 84 is provided to supply a transversal light input to the transparent hypotube providing the pumping wavelength to the doped material. The external light source 84 is provided for coupling light into the transparent hypotube to activate the doped material.

The external light source 84 may be connected to a console 85, as indicated by wire connection or wireless. The console 85 may be part of a control unit or may comprise a control unit that controls the operation of the interventional system and/or the interventional device. The console 85 can also be referred to as control unit.

The interventional device 82 is provided for being inserted into a subject 86, e.g. arranged on a patient support 88. Further, the system 80 may be used in the context of a medical imaging arrangement 90, which is shown as a C-arm based X-ray imaging device.

The term "subject" may also be referred to as individual. The "subject" may further also be referred to as patient, although it is noted that this term does not indicate whether any illness or disease is actually present with the subject.

In an example, the external light source 84 is provided as one of the examples of the external transversal light source 36 described above.

The external light source 84 is connectable to the elongated main body in the region of the transparent hypotube, for example along the transparent hypotube.

In an example, the external light source has an extension in direction of the elongated main body, and the optical energy generating section has a length equal or larger than the extension of the external light source.

The length of the optical energy generating section is determined by the desired degree of insertion freedom (i.e. room for maneuvering) with simultaneous light supply of the sensor, if the light source is arranged to be movable in relation to the main body.

In another example, the light source is non-movable and the length of the optical energy generating section is adapted to the length extension of the external light source.

In an example, the length of the optical energy generating section is approximately 2 cm. In a further example, the length of the optical energy generating section is approximately in the range of 50 cm to 150 cm.

The pumping light from the external light source is provided with a third frequency range. In an option, the third frequency range is different from the first frequency range and/or the second frequency range.

In an example, the third frequency range is adjacent to the first and/or second frequency range without overlapping. In an example, the third frequency range is separated from the first and/or second frequency range by a gap range.

The first frequency can also be referred to as stimulated frequency or generated frequency.

The second frequency can also be referred to as signal frequency or measurement frequency.

The third frequency can also be referred to as pumping frequency or stimulating frequency or initial frequency.

In an example, the external light source 84 is provided as a detachable light source. For example, the external light source 84 can slide along at least a part of the optical energy generation section of the elongated main body.

The light source and the elongated main body are movably in relation to each other. For example, the light source can slide along the elongated main body to allow light input at various locations along the optical energy generation section. In another example, the external light source is temporarily fixed in relation to the subject and the elongated main body can slide in relation to the light source. For example, the light input is provided throughout the sliding motion.

In an option, the external light source is provided integrated with a vascular access port of the interventional device.

As a result, no additional cables or the like are provided that are moved during use of the interventional device.

A vascular access port could be a guiding catheter, hemostatic valve, introducer sheath etc.

A vascular access port could be connected to the patient 86, keeping the external light source in 84 in place while the interventional device 82 is moved.

The access port can be provided with integrated batteries for power supply of the external light source. In another example, the access port is provided with cables for the energy supply. However, the cables would be installed and not moved as long as the access port is in use at the same location.

As an option, an external signal detector 92 is provided that detects light emitted from the light scattering section (see also Fig. 3). The external signal detector 92 may be connected to the console 85, as indicated by wire connection or wireless.

For example, the external signal detector can be provided attached to imaging equipment like the C-arm of an X-ray imaging system, or to lighting equipment in the vicinity of a subject support, e.g. the patient table 88.

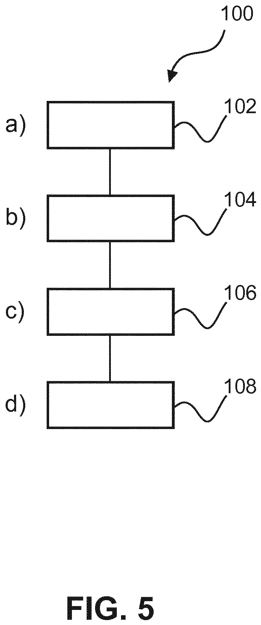

Fig. 5 shows basic steps of an example of a method 100 for supplying optical energy to a sensor of an interventional device.

In a first step 102, also referred to as step a), an interventional device for intravascular application is provided that comprises a longitudinal elongated main body with a distal end and a proximal portion, and a sensor provided near the distal end.

In a second step 104, also referred to as step b), a pumping light with a pumping wavelength is generated and the pumping light is coupled as transversal light input into a hollow shaft provided as a transparent hypotube at the proximal portion, inside which hollow shaft a doped material is provided.

In a third step 106, also referred to as step c), the doped material is stimulated with the light with the pumping wavelength, and thereby light as stimulated emission with a predetermined wavelength is generated.

In a fourth step 108, also referred to as step d), the light is guided by an optical fiber arrangement inside a hollow shaft of the elongated main body to the sensor.

The pumping light can also be referred to as activation light.

In an example, not further shown, the light generated by the optical energy generation section is provided for communicating with the sensor via light modulation. In another example, provided in addition or alternatively, the light generated by the optical energy generation section is provided for supplying the sensor with energy provided by the guided light. In an example, the light is transmitted by an optical fiber arrangement toward the sensor and the light is transformed in electrical energy for powering the sensor.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated, and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. An interventional device (10) configured for at least partial insertion into a body of a subject, the device comprising:
- a longitudinal elongated main body (12) with a distal portion and a proximal portion (16); and
- a sensor (18) provided at the distal portion of the elongated main body;
wherein the elongated main body comprises a hollow shaft (20);
wherein the proximal portion of the main body comprises an optical energy generation section (22), in which the hollow shaft is at least partially provided as a transparent hypotube, and in which a doped material (24) is provided inside the hollow shaft;
wherein the doped material is configured to generate light as stimulated emission with a predetermined wavelength upon the doped material being radiated with a pumping wavelength;
wherein the transparent hypotube is configured to receive light from an external light source as a substantially transversal light input (26) providing the pumping wavelength to the doped material; and
wherein the main body further comprises a light guiding section (28) that comprises an optical fiber arrangement (30) inside the hollow shaft extending from the optical energy generation section toward the sensor for transmitting energy to the sensor.

2. Device according to claim 1, wherein the light generated by the optical energy generation section is provided for:
i) communicating with the sensor via light modulation; and/or
ii) supplying the sensor with energy provided by the transmitted light.

3. Device according to claim 1 or 2, wherein it is further provided:
- a first mirror element (32) at a distal transition portion of the optical energy generation section with the doped material to the optical fiber arrangement in the hollow shaft of the light guiding section; and
- a second mirror element (34) at a proximal end portion of the optical energy generation section with the doped material;
wherein the first mirror element is partly reflective for the generated light with a first reflective grade; and wherein the second mirror element is reflective for the generated light with a second reflective grade being higher than the first reflective grade.

4. Device according to claim 1, 2 or 3, wherein the sensor provides measurement results as optical signals that are guided by the optical fiber arrangement; and
wherein a light scattering section (50) is provided at the proximal portion of the device that comprises a light scattering material (52) to provide the optical signals as scattered light (54) for detection by an external signal detector (56).

5. Device according to one of the preceding claims, wherein the light generated by the stimulated emission by the doped material is provided with a first frequency range, and the optical signals from the sensor are provided with a second frequency range; and
wherein the first frequency range is different from the second frequency range.

6. Device according to one of the preceding claims, wherein the interventional device is at least one of the group of a guidewire, a catheter and a needle; and
wherein the sensor is at least one of the group of a pressure sensor, an imaging sensor (ultrasound, OCT), a temperature sensor, a pH-sensor, a biomarker sensor, a chemical sensor, a flow volume sensor, a light absorption sensor, a light scattering sensor and a flow velocity sensor.

7. Device according to one of the preceding claims, wherein the distal portion of the device contains a photodiode and an electrical sensor, wherein the photodiode transfers optical energy into electrical energy to power the sensor.

8. Device according to one of the preceding claims, wherein the doped material is of rare earth elements comprising erbium and ytterbium ions.

9. An interventional system (50), comprising:
- an interventional device (52) according to one of the preceding claims; and
- an external light source (54);
wherein the external light source is provided to supply a substantially transversal light input to the transparent hypotube providing the pumping wavelength to the doped material.

10. System according to claim 9, wherein the light source is provided as a detachable light source.

11. System according to claim 9 or 10, wherein the external light source can slide along at least a part of the optical energy generation section of the elongated main body.

12. System according to one of the claims 9 to 11, wherein the external light source comprises a plurality of pumping light emitting diodes that are arranged at least along a part of circumferential section of the transparent hypotube; and
wherein a shielding (40) is provided that optically shields the outside from the light of the pumping light emitting diodes.

13. System according to one of the claims 9 to 12, wherein the external light source is provided integrated with a vascular access port of the interventional device.

14. System according to one of the claims 9 to 13, wherein an external signal detector (92) is provided that detects light emitted from the light scattering section.

15. A method (100) for supplying energy to a sensor of an interventional device, the method comprising the following steps:
a) providing (102) an interventional device configured for at least partial insertion into a body of a subject, the device comprising: a longitudinal elongated main body with a distal portion and a proximal portion; and a sensor provided at the distal portion;
b) generating (104) a pumping light with a pumping wavelength and coupling the pumping light as substantially transversal light input into a hollow shaft provided as a transparent hypotube at the proximal portion, inside which hollow shaft a doped material is provided;
c) stimulating (106) the doped material with the light with the pumping wavelength and thereby generating light as stimulated emission with a predetermined wavelength; and
d) transmitting (108) the light by an optical fiber arrangement inside a hollow shaft of the elongated main body toward the sensor for supplying energy to the sensor.
